# EUROPÄISCHE PATENTSCHRIFT

(11) **EP 1 001 265 B1**
(45) Veröffentlichungstag und Bekanntmachung des Hinweises auf die Patenterteilung: **17.09.2003**
(21) Anmeldenummer: 99122308.2
(22) Anmeldetag: 09.11.1999
(51) Int. Cl.: G01N 33/04, G01N 1/10, B01L 3/00

(54) **Probenflasche**
Sampling bottle
Bouteille d'échantillonnage

(30) Priorität: 09.11.1998 DE 29819987 U
(43) Veröffentlichungstag der Anmeldung: 17.05.2000
(73) Patentinhaber: Bartec GmbH, 97980 Bad Mergentheim (DE)
(72) Erfinder: Böhm, Alfred, D-94234Viechtach (DE); Barlian, Reinhold A., D-97980 Bad Mergentheim (DE)
(74) Vertreter: Heim, Hans-Karl, Dipl.-Ing.

(56) Entgegenhaltungen:
- EP-A- 0 644 503
- DE-A- 4 135 420
- DE-C- 341 873
- NL-C- 1 000 084

## Beschreibung

Die Erfindung betrifft eine Probenflasche, insbesondere eine Milchprobenflasche, gemäß dem Oberbegriff des Anspruchs 1.

Eine gattungsgemäße Milchprobenflasche ist in der EP O 411 274 B1 sowie in dem Datenblatt "Milchprobenflasche Typ 6845-x" der ULTRAKUST Electronik GmbH beschrieben.

Die Identifizierung der einzelnen Probenflaschen erfolgt durch Auslesen der Daten auf einem Barcode-Etikett, welches in der Regel etwa im Mittelteil des Flaschenkörpers angebracht ist. Durch Anordnung des Barcode-Etiketts im Bereich einer Einschnürung des Flaschenkörpers soll verhindert werden, dass sich das Barcode-Etikett beim Transport, beispielsweise aufgrund eines Aneinanderreibens der Flaschen, löst oder beschädigt wird.

Aus der EP O 644 503 A1 und DE 41 35 420 A1 sind Probenflaschen für Milchsammelwagen bekannt, welche am Umfang mit einem Barcode bzw. mit einem Barcode-Klebeetikett versehen sind, deren Daten von einem Lesekopf einer Identifikationseinrichtung ausgelesen werden können.

In der DE 34 15 873 A1 ist eine Transport- und Kodiervorrichtung für Milchprobenflaschen beschrieben, welche jeweils einen magnetischen Datenträger auf ihrer Umfangswand aufweisen. Der magnetische Datenträger wird in der Kodiervorrichtung mit lieferantenspezifischen Daten kodiert, wobei gleichzeitig das Befüllen der jeweiligen Milchprobenflasche erfolgt.

Es hat sich jedoch gezeigt, dass die Barcodes bzw. Barcode-Etiketten und magnetischen Datenträger nach längerem Gebrauch der Probenflaschen verschmutzen und auch zerkratzt werden, wodurch es schwierig oder unmöglich wird, deren Inhalt auszulesen. Ein weiterer Nachteil der Barcode-Etiketten ist darin zu sehen, dass diese nur ein Auslesen der Daten, jedoch kein Beschreiben, z.B. zur Aktualisierung der Daten, zulassen.

Aus der NL 1000084 C ist eine Einrichtung zur Entnahme von Milchproben bekannt, bei welcher Milchprobenflaschen mit einem elektromagnetischen Bauteil am Boden benutzt werden. Die Milchprobenflaschen weisen die Form eines Reagenzglases auf, und jeweils fußseitig angeordnete elektromagnetische Identifikationseinrichtungen können ausgelesen und beschrieben werden. Diese bekannten Milchprobenflaschen sind für die in der Regel zum Transport und zur Indentifikation der Probenflaschen eingesetzten Rundmagazine bzw. Kassetten, welche antreibbare magnetische Böden aufweisen, nicht geeignet.

Der Erfindung liegt die **Aufgabe** zugrunde, eine Probenflasche, insbesondere eine Milchprobenflasche, zu schaffen, welche außerordentlich einfach und kostengünstig mit einer les- und beschreibbaren Einrichtung für Daten, insbesondere für Identifikationsdaten und Produkt- und Lieferantenspezifische Daten, versehen und in Transport- und Identifikationsvorrichtungen mit magnetischen Böden eingesetzt werden kann.

Erfindungsgemäß wird die Aufgabe durch die Merkmale des Anspruchs 1 gelöst. Zweckmäßige und vorteilhafte Ausgestaltungen sind in den Unteransprüchen und in der Figurenbeschreibung enthalten.

Die Erfindung geht von der Überlegung aus, als ein elektronisch beschreib- und lesbares Bauteil einen TAG für die Identifizierung und als Datenträger für veränderbare Daten der Probenflaschen bzw. Proben einzusetzen und eine sichere, geschützte und außerordentlich einfache und kostengünstige Positionierung des TAG durch eine Vertiefung am Flaschenumfang sowohl an bereits hergestellten und verwendeten als auch noch zu fertigenden Probenflaschen zu erreichen.

Elektronische Bauteile mit digitalen Speichereinrichtungen, in denen die zu generierenden und abzugebenden Daten enthalten sind und welche als Lese- oder Schreib-Lesespeicher ausgebildet sein können, werden bereits auf unterschiedlichen Gebieten eingesetzt.

TAGs, welche auch als Transponder bezeichnet werden können, sind für den Einsatz an Probenflaschen besonders geeignet, weil die Daten- und Energieübertragung berührungslos, beispielsweise induktiv, erfolgen kann. Als Schreib-Leseeinrichtungen können TAG-Reader bzw. Schreib-Lesestifte eingesetzt werden, welche die ausgelesenen Daten an eine Steuereinrichtung und/oder eine Datenbank weiterleiten können.

Mit der erfindungsgemäßen Probenflasche wird die Möglichkeit geschaffen, eine außerordentlich sichere elektronische Identifizierung der Probenflasche zu erreichen und darüber hinaus auch variable Daten, insbesondere Produktdaten und für den Lieferanten spezifische Daten, zu speichern und zu aktualisieren. Der besondere Vorteil besteht darin, dass die Datenspeicherung, -übertragung und -aktualisierung flaschenseitig besonders kostengünstig gestaltet werden kann. Die Fertigungskosten für eine mit einem TAG ausgesrüstete Probenflasche können trotz der z.Zt. noch relativ kostenaufwendigen TAGs in Grenzen gehalten werden.

Es ist besonders vorteilhaft, dass sowohl die Umrüstung der im Gebrauch befindlichen Milchprobenflaschen als auch die Herstellung neuer und erfindungsgemäß mit wenigstens einer Vertiefung versehenen Probenflaschen außerordentlich einfach und mit relativ niedrigen Kosten durchgeführt werden können.

Die Vertiefung zur Aufnahme eines TAG ist zweckmäßigerweise muldenförmig ausgebildet und entsprechend dem jeweiligen TAG dimensioniert.

Es ist sinnvoll, einen TAG und ein Barcode-Etikett auf einer Probenflasche anzubringen. Das Barcode-Etikett kann in der herkömmlichen Weise ausgebildet oder gegebenenfalls auch modifiziert werden, damit es zusätzlich als Schutz für den TAG dienen kann.

Die TAG-Vertiefung kann grundsätzlich in jedem Bereich des Flaschenumfangs ausgebildet werden. Es ist vorteilhaft, wenn der TAG im Bereich des Barcode-Etiketts angebracht und z.B. von diesem abgedeckt wird. Das Barcode-Etikett ist üblicherweise im Bereich einer umlaufenden Einschnürung des Flaschenkörpers angeklebt. Die nach innen gerichtete Vertiefung für den TAG kann dann ebenfalls geschützt im Bereich dieser Einschnürung ausgebildet werden.

Die Unterbringung des Chips auf der Milchprobenflasche im Bereich des Barcode-Etikettes ist mit dem Vorteil verbunden, dass die bisher bekannte Gerätetechnik für Analyse und Flaschenbehandlung weiter eingesetzt werden kann.

Es ist vorteilhaft, dass die Probenflasche bis auf die Vertiefung wie üblich ausgebildet und einen Kunststoff-Flaschenkörper mit einer Öffnung zur Aufnahme eines Verschlussstopfens und einen topf- oder ringförmigen Metallsokkel aufweisen kann.

Dass eine Vertiefung für wenigstens einen TAG auch nachträglich in eine Probenflasche eingebracht werden kann, ist ein weitere Vorteil. Die in einer besonders großen Zahl im Umlauf befindlichen Milchprobenflaschen können auf diese Weise zur Aufnahme eines TAG oder eines ähnlichen elektronischen Bauteils bzw. Chips einfach und kostengünstig umgerüstet werden.

Besonders einfach kann eine Vertiefung mit Hilfe eines stempelförmigen Werkzeugs nach einer lokalen Erwärmung des Flaschenkörpers hergestellt werden. Diese effiziente Herstellung einer Vertiefung kann darüber hinaus auch dann angewendet werden, wenn ein elektronisches Bauteil bzw. TAG ausgewechselt werden soll und das neue Bauteil eine abweichende Form und Dimensionierung aufweist.

Grundsätzlich kann die Ausbildung wenigstens einer TAG-Vertiefung in den Herstellungsprozess für neue Probenflaschen integriert werden, weil die Flaschenform, Wandstärken usw. nicht verändert werden müssen.

Zweckmäßigerweise sollte die Vertiefung muldenförmig und insbesondere innenseitig ohne scharfe Kanten bzw. Hinterschneidungen ausgebildet sein, um eine problemlose Reinigung der Probenflasche gewährleisten zu können.

Eine bevorzugte Ausbildung sieht die Anordnung der TAG-Vertiefung unterhalb eines Barcode-Etiketts vor. Der TAG kann beispielsweise eingeschweißt, eingeklebt und/oder vergossen werden. Um den Leseradius des darüber angeordneten Barcode-Etiketts aufrechtzuerhalten, sollte der TAG formschlüssig mit der Außenkontur des Flaschenkörpers abschließen, was vorteilhaft mit einem Deckel und/oder einer Vergussmasse erreicht werden kann.

Bei Ausrüstung der Probenflaschen mit einem TAG und einem Barcode-Etikett können die für den Barcode verwendeten Auslesestationen beibehalten werden und müssen nur zusätzlich mit einem TAG-Reader oder einer Schreib-Leseeinrichtung für den TAG versehen werden.

Obwohl die Ausbildung der Vertiefung in dem zylinderförmigen Bereich des Flaschenkörpers und insbesondere im Bereich der Einschnürung die bevorzugte Ausbildungsvariante darstellt, liegt es im Rahmen der Erfindung, eine TAG-Vertiefung auch in einem oberen Schrägteil des Flaschenkörpers anzuordnen. Die TAG-Vertiefung könnte hier auch in einer nach außen gerichteten Ausstülpung ausgebildet werden. Die Innenwandung der Probenflasche wäre dann wie bisher üblich glatt ausgebildet, die Positionierung des TAG über die nach außen gerichtete Ausformung jedoch leicht zu erkennen.

Eine Anordnung des TAG wäre grundsätzlich auch im Bereich des Metallsockels denkbar.

Es ist besonders günstig, den TAG geschützt und unsichtbar unterhalb des Barcode-Etiketts anzuordnen. Eine besonders vorteilhafte Ausbildung sieht eine integrierte Anordnung des TAG in dem Barcode-Etikett vor. Daneben kann der TAG auch mit einer anderen Abdeckung geschützt werden. Das Barcode-Etikett kann dann außerhalb des TAG, beispielsweise an einem gegenüberliegenden Bereich angeordnet werden.

Wenn das Barcode-Etikett derart dimensioniert ist, dass es über den gesamten Umfang des Flaschenkörpers reicht und sich mit Endbereichen überlappt, wird gleichzeitig eine besonders gute Haftung des Etiketts erreicht. Der Barcode selbst nimmt dann nur einen Teil des Etiketts ein und deckt vorteilhaft einen TAG oder ähnlichen Schreib-Lese-Chip direkt oder zusätzlich zu einer Abdeckung ab.

Die Erfindung wird nachstehend anhand einer Zeichnung weiter erläutert; in dieser zeigen:
- Fig. 1: eine Ansicht einer erfindungsgemäßen Probenflasche mit einem TAG;
- Fig. 2: eine Ansicht einer erfindungsgemäße Probenflasche mit einem Barcode-Etikett;
- Fig. 3: einen Querschnitt einer erfindungsgemäßen Probenflasche im Bereich eines TAG;
- Fig. 4 bis 6: Querschnitte von erfindungsgemäßen Probenflaschen mit einem unterschiedlich angeordneten TAG und Barcode-Etikett und
- Fig. 7: eine Ansicht einer erfindungsgemäßen Probenflasche mit einem TAG und einer Abdeckung.

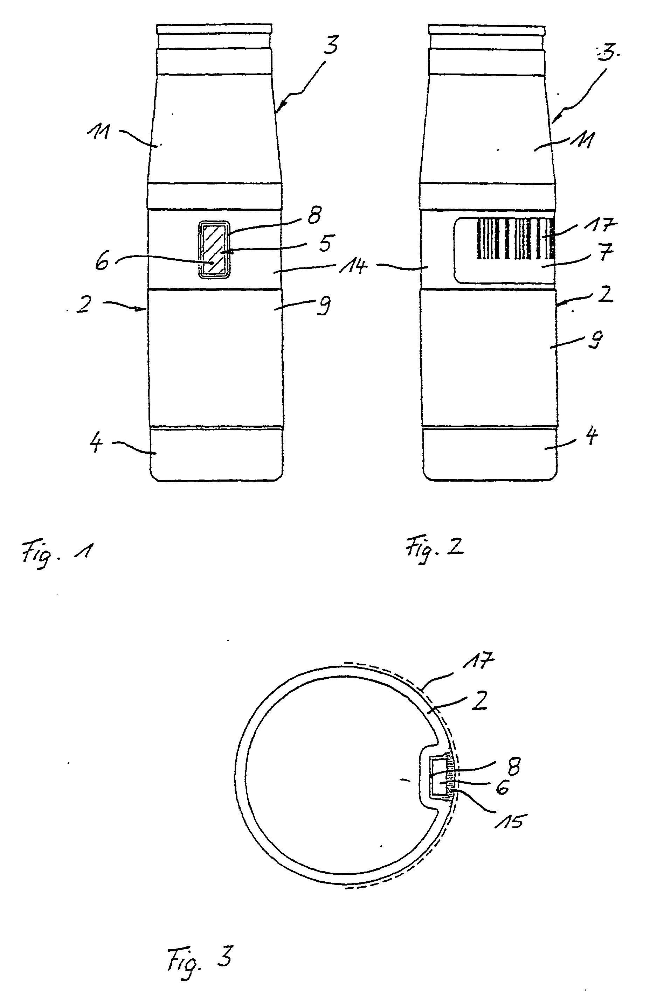

Figur 1 zeigt eine Probenflasche 3, welche als Milchprobenflasche eingesetzt wird. Die Probenflasche 3 besteht aus. einem Flaschenkörper 2 und einem Metallsockel 4, welcher als ein magnetisch weicher Stahltopf ausgebildet ist und einen Transport der Probenflasche 3 durch Magnetketten oder Magnetplatten in Rundmagazinen ermöglicht.

Der Flaschenkörper 2 ist aus Kunststoff gefertigt und kann beispielsweise aus einem Polypropylen-Derivat bestehen. In Form und Material entspricht die Probenflasche 3 den herkömmlichen und in einer großen Zahl bei der Milchprobenentnahme eingesetzten Probenflaschen.

Um eine sichere automatische Probenflaschenidentifizierung und darüber hinaus auch eine Speicherung und Auswertung variabler Daten, z.B. der gesamten Produkt- und/oder Lieferanteninformationen mit der Probenflasche zu erreichen, ist zusätzlich zu einem an sich bekannten Barcode-Etikett 7 (siehe Fig. 2) ein elektronisches Bauteil 6 bzw. ein Chip angeordnet. Dabei handelt es sich um einen schreib- und auslesbaren TAG 6, bei welchem die Daten- und Energieübertragung berührungslos erfolgt.

Bei den in den Figuren gezeigten Probenflaschen 3 ist der TAG 6 wie das Barcode-Etikett 7 (Figuren 2, 4 bis 7) an einem zylinderförmigen Bereich 9 des Flaschenkörpers 2 im Bereich einer Einschnürung 14 angeordnet. Um eine Zerstörung oder Beschädigung des TAG 6 zu vermeiden sowie aus Gründen einer effektiven Probenflaschenherstellung und -umrüstung, wird der TAG 6 in einer Vertiefung 8 des Flaschenkörpers 2 angeordnet. Figur 1 und der Querschnitt gemäß Fig. 3 verdeutlichen, dass die Vertiefung 8 in Form und Dimensionierung dem eingesetzten TAG 6 angepasst und eine vollständige Aufnahme des TAG 6 ohne Überstand gewährleistet ist.

Figur 1 zeigt die Probenflasche 3 von vorn und ohne Barcode-Etikett 7. In Fig. 2 ist nur das Etikett 7 mit dem Barcode 17 sichtbar, welches über dem TAG 6 angeordnet ist, wodurch dieser zusätzlich geschützt und unsichtbar ist. Um die Lesbarkeit des Barcodes 17 auch im Bereich des TAG 6 zu gewährleisten, ist der TAG 6 außerdem mit einem formschlüssigen Verschluss 15 (Fig. 3) abgedeckt, dessen Außenkontur der Wölbung des Flaschenkörpers 2 und dem Leseradius des Barcode-Etiketts 7 entspricht.

Die Querschnittsdarstellungen der Figuren 3 bis 6 lassen erkennen, dass die Ausformung der Vertiefung 8 in den Herstellungsprozess des Flaschenkörpers 2 integriert werden kann, jedoch auch an bereits hergestellten und benutzten Probenflaschen 3 wenigstens eine Vertiefung 8 nächträglich eingebracht werden kann.

Grundsätzlich könnte eine TAG-Vertiefung 8 auch im Bereich eines Schrägteils 11 des Flaschenkörpers 2 oder im Bereich des Metallsockels 4 ausgebildet werden. In bezug auf die Schreib- und Leseeinrichtungen (nicht dargestellt) empfiehlt sich jedoch eine Positionierung des TAG 6 in einem Mittelbereich des Flaschenkörpers 2, wie es beispielhaft in den Figuren 1, 2 und 7 dargestellt ist.

Zur übersichtlicheren Darstellung wurde in Fig. 3 nur der Barcode 17, jedoch nicht das Barcode-Etikett 7 dargestellt. Der Flaschenkörper 2 gemäß Fig. 4 ist mit einem Barcode-Etikett 7 versehen, welches im Bereich des TAG 6 den Barcode 17 aufweist.

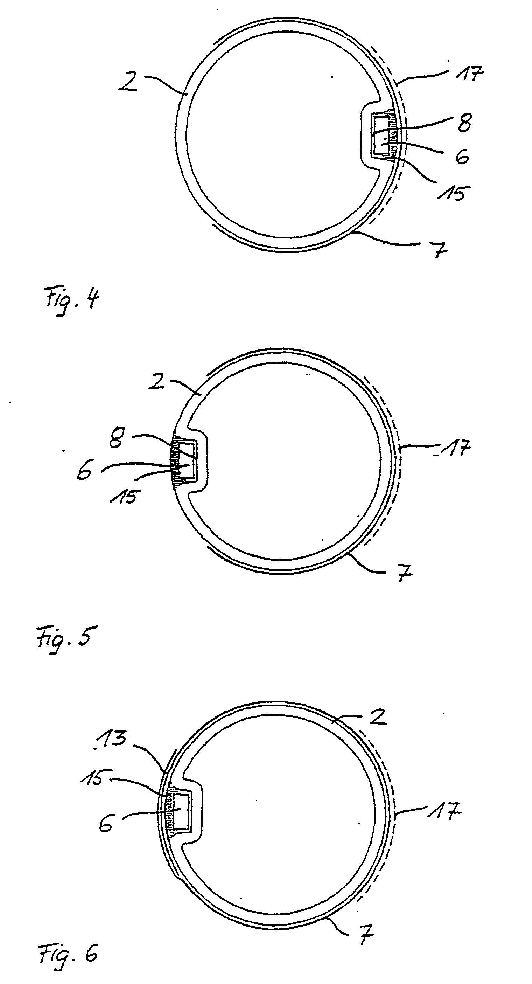

Bei dem Flaschenkörper 2 der Fig. 5 ist die TAG-Vertiefung 8 außerhalb des Barcode-Etiketts 7 und nahezu diametral zum Barcode 17 ausgebildet. Der TAG 6 wird hier von einer Vergussmasse 15 als einem formschlüssigen Verschluss abgedeckt und durch diesen geschützt.

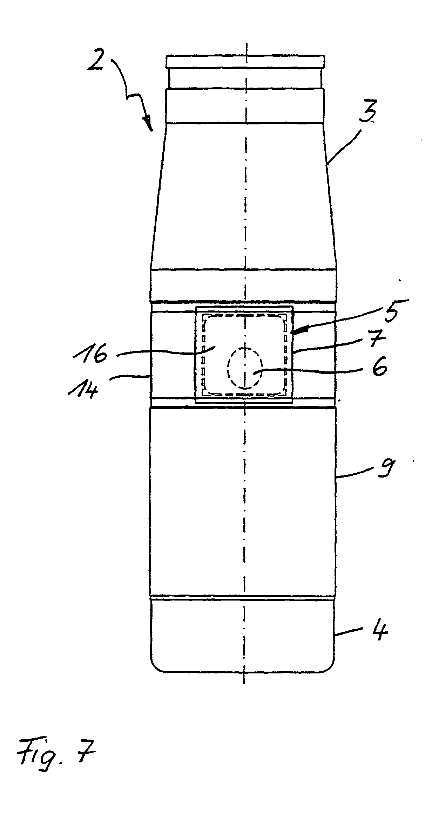

Die Ausbildungsvariante nach Fig. 6 zeigt ein Barcode-Etikett 7, welches im Bereich der Vertiefung 8 und des TAG 6 einen sich überlappenden Bereich 13 aufweist. Der Barcode 17 ist nahezu gegenüber dem TAG 6 angeordnet. Der sich überlappende Bereich 13 des Barcode-Etiketts 7 sorgt nicht nur für einen zusätzlichen Schutz des mit einer Vergussmasse 15 abgedeckten TAG 6, sondern bewirkt auch eine bessere Haftung des Barcode-Etiketts 7 an der Probenflasche 3.

Die Probeflasche gemäß Fig. 7 ist im Bezug auf den Kunststoff-Flaschenkörper 2 mit seinem zylinderförmigen Bereich 9 und dem Metallsockel 4 grundsätzlich identisch zu den Probenflaschen 3 der vorangegangenen Figuren ausgebildet. Identische Merkmale sind mit gleichen Bezugszeichen versehen.

Im Bereich einer Einschnürung 14 ist ein TAG 6 durch Verschweißen am Flaschenkörper 2 angeordnet und mit einer Abdeckung 16, welche als ein Deckel ausgebildet ist, abgedeckt. Über der Abdeckung 16 ist ein Barcode-Etikett 7 aufgeklebt, wobei in diesem Beispiel der Barcode nicht dargestellt ist.

Der TAG 6 als elektronisches Bauteil mit einem Schreib-Lese-Speicher ermöglicht es, dass die Probenflasche nicht nur sicher identifizierbar, sondern auch zur Speicherung der gesamten Produkt- und Lieferanteninformationen als Datenträger verwendet werden kann. Die Aufzeichnung der Informationen kann z.B. in Form eines BCD-Codes numerisch oder alphanumerisch erfolgen. Dabei kann es vorteilhaft sein, wenn bestimmte Informationen normiert abgelegt werden. Es kann beispielsweise von Vorteil sein, dass die Aufzeichnung von Zusatzinformationen anwenderspezifisch in verbleibenden freien Pages des Transponders erfolgt. Eine Zuordnung zu speziellen Datensatzformaten ist nicht erforderlich. Die Formatzuordnung kann entsprechend den verschiedenen Datenformaten durch die jeweilige Lesesoftware realisiert werden. Als Produkt- und Lieferanteninformationen sind insbesondere die Milchart, z.B. Rohmilch der verschiedenen Güteklassen oder Biomilch, sowie Uhrzeit, Datum, Fahrzeugnummer, Fahrernummer, Betriebsnummer, Tournummer, außerdem die Probenart, beispielsweise Inhaltsstoffprobe, Lieferantenprobe oder Bakterienprobe, Handprobe, Laborprobe etc., sowie die Milchmenge, Milchtemperatur, Probenfachtemperatur, Probenflaschenvolumen, Probentyp Labor, vorzusehen.

Indem die erfindungsgemäße Probenflasche mit einem elektronischen Lesespeicher, bevorzugt jedoch mit einem Schreib-Lesespeicher ausgerüstet ist oder umgerüstet wird, sind die Identifizierung der Probenflaschen und die Datenspeicherung und Datenübertragung besonders sicher gewährleistet.

Für die Herstellung der erfindungsgemäßen Probenflasche ist es vorteilhaft, dass der Schreib-Lese-Chip in das Barcode-Etikett integriert und mit diesem auf dem Flaschenkörper der Probenflasche aufgebracht werden kann.

## Patentansprüche

1. Probenflasche, insbesondere Milchprobenflasche, mit einem Flaschenkörper (2), welcher eine elektronische Identifikationseinrichtung (5) mit einem Lesespeicher oder einem Schreib-Lese-Speicher für Identifikationsdaten und/oder für variable Produkt- und/oder Lieferantendaten aufweist,
**dadurch gekennzeichnet,**
**dass** als elektronische Identifikationseinrichtung (5) ein TAG (6) angeordnet ist,
**dass** der Flaschenkörper (2) im Bereich des Flaschenumfangs mit einer Vertiefung (8) versehen ist und
**dass** der TAG (6) in der Vertiefung (8) aufgenommen ist.

2. Probenflasche nach Anspruch 1,
**dadurch gekennzeichnet,**
**dass** die Vertiefung (8) muldenförmig und komplementär zu dem aufzunehmenden TAG (6) in dem Flaschenkörper (2) ausgebildet ist.

3. Probenflasche nach Anspruch 1 oder 2, mit einem Metallsockel (4),
**dadurch gekennzeichnet,**
**dass** die Vertiefung (8) in einem zylinderförmigen Bereich (9) des Flaschenkörpers (2) oberhalb des Metallsockels (4) ausgebildet ist.

4. Probenflasche nach einem der vorhergehenden Ansprüche,
**dadurch gekennzeichnet,**
**dass** zusätzlich ein Barcode-Etikett (7) an dem Flaschenkörper (2) angebracht ist.

5. Probenflasche nach einem der vorhergehenden Ansprüche,
**dadurch gekennzeichnet,**
**dass** die Ausbildung der Vertiefung (8) in die Herstellung des Flaschenkörpers (2) integriert ist.

6. Probenflasche nach einem der Ansprüche 1 bis 4,
**dadurch gekennzeichnet,**
**dass** die Vertiefung (8) nachträglich an dem fertigen Flaschenkörper (2) nach einer lokalen Erwärmung mit Hilfe eines stempelförmigen Werkzeugs herstellbar ist.

7. Probenflasche nach einem der vorhergehenden Ansprüche,
**dadurch gekennzeichnet,**
**dass** der TAG (6) in der Vertiefung (8) formschlüssig angeordnet und eingeschweißt, verklebt oder vergossen ist.

8. Probenflasche nach einem der Ansprüche 4 bis 7,
dass das Barcode-Etikett (7) unter Abdeckung des eingeschweißten, vergossenen oder verklebten TAG (6) aufgebracht, insbesondere aufgeklebt ist.

9. Probenflasche nach einem der Ansprüche 4 bis 7,
**dadurch gekennzeichnet,**
**dass** der TAG (6) und das Barcode-Etikett (7) an unterschiedlichen Bereichen des Flaschenkörpers (2) angeordnet sind.

10. Probenflasche nach Anspruch 9,
**dadurch gekennzeichnet,**
**dass** das Barcode-Etikett (7) gegenüber der den TAG (6) aufnehmenden Vertiefung (8) angeordnet ist.

11. Probenflasche nach einem der Ansprüche 4 bis 9,
**dadurch gekennzeichnet,**
**dass** das Barcode-Etikett (7) unter Abdeckung der Vertiefung (8) und des darin aufgenommenen TAG (6) um den Flaschenkörper (2) reicht und einen überlappenden Bereich (13) aufweist.

12. Probenflasche nach einem der Ansprüche 3 bis 11,
**dadurch gekennzeichnet,**
**dass** die Vertiefung (8) in einer Einschnürung (14) des zylinderförmigen Bereichs (9) des Flaschenkörpers (2) ausgebildet ist.

13. Probenflasche nach Anspruch 1 oder 2,
**dadurch gekennzeichnet,**
**dass** die Vertiefung (8) in einem Schrägteil (11) des Flaschenkörpers (2) ausgebildet ist.

14. Probenflasche nach einem der vorhergehenden Ansprüche,
**dadurch gekennzeichnet,**
**dass** der TAG (6) in das Barcode-Etikett (6) integriert und mit diesem auf dem Flaschenkörper (2) fixierbar ist.

15. Probenflasche nach Anspruch 1 oder 2,
**dadurch gekennzeichnet,**
**dass** die Vertiefung (8) in einem Bodenbereich (12) des Flaschenkörpers (2) ausgebildet ist.

16. Probenflasche nach einem der Ansprüche 2 bis 15,
**dadurch gekennzeichnet,**
**dass** der Flaschenkörper (2) aus einem Polypropylen-Derivat und der Metallsockel (4) aus einem magnetisch weichen Stahl gefertigt ist und dass der Flaschenkörper (2) mit dem Metallsockel (4), welcher insbesondere topfförmig ausgebildet ist, verpresst ist.

## Claims

1. Sampling bottle, particularly milk sampling bottle, having a bottle body (2), which is provided with an electronic identification device (5) with a read-only memory or a random access memory for identification data and/or for variable product and/or supplier data,
**characterized in that**
the electronic identification device (5) is constituted by a tag (6), that the bottle body (2) is provided in the area of the bottle circumference with a depression (8) and that the tag (6) is received in the depression (8).

2. Sampling bottle according to claim 1,
**characterized in that**
the depression (8) is trough-shaped and constructed in complementary manner to the tag (6) to be received in the bottle body (2).

3. Sampling bottle according to claim 1 or 2 having a metal base (4),
**characterized in that**
the depression (8) is constructed in a cylindrical area (9) of the bottle body (2) above the metal base (4).

4. Sampling bottle according to one of the preceding claims,
**characterized in that**
additionally a bar code label (7) is applied to the bottle body (2).

5. Sampling bottle according to one of the preceding claims,
**characterized in that**
the construction of the depression (8) is integrated into the manufacture of the bottle body (2).

6. Sampling bottle according to one of the claims 1 to 4,
**characterized in that**
the depression (8) can be made subsequently on the finished bottle body (2) following local heating with the aid of a punch-like tool.

7. Sampling bottle according to one of the preceding claims,
**characterized in that**
the tag (6) is positively placed in the depression (8) and is welded in, stuck or cast.

8. Sampling bottle according to one of the claims 4 to 7,
**characterized in that**
the bar code label (7) is applied and particularly stuck on covering the welded in, cast or stuck tag (6).

9. Sampling bottle according to one of the claims 4 to 7,
**characterized in that**
the tag (6) and bar code label (7) are located in different areas of the bottle body (2).

10. Sampling bottle according to claim 9,
**characterized in that**
the bar code label (7) faces the depression (8) receiving the tag (6).

11. Sampling bottle according to one of the claims 4 to 9,
**characterized in that**,
whilst covering the depression (8) and the tag (6) received therein, the bar code label (7) extends around the bottle body (2) and has an overlapping area (13).

12. Sampling bottle according to one of the claims 3 to 11,
**characterized in that**
the depression (8) is constructed in a constriction (14) of the cylindrical area (9) of the bottle body (2).

13. Sampling bottle according to claim 1 or 2,
**characterized in that**
the depression (8) is formed in an inclined part (11) of the bottle body (2).

14. Sampling bottle according to one of the preceding claims,
**characterized in that**
the tag (6) is integrated into the bar code label (7) and is fixable therewith on the bottle body (2).

15. Sampling bottle according to claims 1 or 2,
**characterized in that**
the depression (8) is formed in a bottom area (12) of the bottle body (2).

16. Sampling bottle according to one of the claims 2 to 15,
**characterized in that**
the bottle body (2) is made from a polypropylene derivative and the metal base (4) from a magnetic, mild steel and that the bottle body (2) is pressed with the metal base (4), which in particular has a cup-shaped construction.

## Revendications

1. Bouteille d'échantillonnage notamment bouteille d'échantillonnage de lait, comportant un corps de bouteille (2) muni d'une installation d'identification électronique (5) avec une mémoire de lecture ou une mémoire lecture/enregistrement pour des données d'identification et/ou pour des données variables concernant le produit et/ou le fournisseur,
**caractérisée en ce que**
l'installation d'identification électronique (5) est une étiquette (6),
le corps (2) de la bouteille comporte au niveau de la périphérie de la bouteille, une cavité (8), et
l'étiquette (6) est logée dans la cavité (8).

2. Bouteille d'échantillonnage selon la revendication 1,
**caractérisée en ce que**
la cavité (8) est en forme d'auge et elle est complémentaire à l'étiquette (6) à recevoir dans le corps (2) de la bouteille.

3. Bouteille d'échantillonnage selon la revendication 1 ou 2, comportant un socle métallique (4),
**caractérisée en ce que**
la cavité (8) est réalisée dans une zone cylindrique (9) du corps (2) de la bouteille au-dessus du socle métallique (4).

4. Bouteille d'échantillonnage selon l'une des revendications précédentes,
**caractérisée en ce que**
le corps de bouteille (2) comporte en outre une étiquette à code barre (7).

5. Bouteille d'échantillonnage selon l'une des revendications précédentes,
**caractérisée en ce que**
la réalisation de la cavité (8) est intégrée dans la fabrication du corps de bouteille (2).

6. Bouteille d'échantillonnage selon l'une des revendications 1 à 4,
**caractérisée en ce que**
la cavité (8) est réalisée ultérieurement dans le corps de bouteille terminé (2) après échauffement local en utilisant un outil en forme de poinçon.

7. Bouteille d'échantillonnage selon l'une des revendications précédentes,
**caractérisée en ce que**
l'étiquette (6) est placée dans la cavité (6) suivant une liaison de forme et elle est soudée, collée, ou coulée.

8. Bouteille d'échantillonnage selon les revendications 4 à 7,
**caractérisée en ce que**
l'étiquette à code barre (7) est appliqué en recouvrant l'étiquette (6) soudée, collée ou coulée et notamment cette étiquette à code barre est collée.

9. Bouteille d'échantillonnage selon l'une des revendications 4 à 7,
**caractérisée en ce que**
l'étiquette (6) et l'étiquette à code barre (7) sont prévues dans des zones différentes du corps de bouteille (2).

10. Bouteille d'échantillonnage selon la revendication 9,
**caractérisée en ce que**
l'étiquette à code barre (7) est prévue en regard de la cavité (8) recevant l'étiquette (6).

11. Bouteille d'échantillonnage selon l'une des revendications 4 à 9,
**caractérisée en ce que**
l'étiquette à code barre (7) entoure le corps (2) de la bouteille en couvrant la cavité (8) et l'étiquette (6) logée dans celle-ci et l'étiquette à code barre comporte une zone de chevauchement (13).

12. Bouteille d'échantillonnage selon l'une des revendications 3 à 11,
**caractérisée en ce que**
la cavité (8) est réalisée dans une partie rétrécie (14) de la zone cylindrique (9) du corps de bouteille (2).

13. Bouteille d'échantillonnage selon la revendication 1 ou 2,
**caractérisée en ce que**
la cavité (8) est réalisée dans une partie inclinée (11) du corps de bouteille (2).

14. Bouteille d'échantillonnage selon l'une quelconque des revendications précédentes,
**caractérisée en ce que**
l'étiquette (6) est intégrée dans l'étiquette à code barre (7) et est fixée avec celle-ci au corps de bouteille (2).

15. Bouteille d'échantillonnage selon l'une des revendications 1 ou 2,
**caractérisée en ce que**
la cavité (8) est réalisée dans la zone de fond (12) du corps de bouteille (2).

16. Bouteille d'échantillonnage selon l'une des revendications 2 à 15,
**caractérisée en ce que**
le corps de bouteille (2) est fabriqué en un dérivé de polypropylène et le socle métallique (4) en un acier doux magnétique, et
le corps de bouteille (2) est pressé avec le socle métallique (4) notamment réalisé en forme de pot.
